# EUROPÄISCHE PATENTSCHRIFT

(11) **EP 3 329 946 B1**
(45) Veröffentlichungstag und Bekanntmachung des Hinweises auf die Patenterteilung: **05.12.2018**
(21) Anmeldenummer: 17001550.7
(22) Anmeldetag: 18.09.2017
(51) Int. Cl.: A61M 1/00

(54) **MEDIZINISCHER SAUGREGULATOR**
MEDICAL VACUUM REGULATOR
RÉGULATEUR DE SUCCION MÉDICAL

(30) Priorität: 30.11.2016 DE 102016014241
(43) Veröffentlichungstag der Anmeldung: 06.06.2018
(73) Patentinhaber: Karl Storz SE & Co. KG, 78532 Tuttlingen (DE)
(72) Erfinder: Teufel, Felix, 78532 Tuttlingen (DE); Kreidler, Bodo, 78532 Tuttlingen (DE)
(74) Vertreter: Hofmeister, Frank

(56) Entgegenhaltungen:
- EP-A1- 0 007 602
- US-A1- 2009 228 030

## Beschreibung

Die Erfindung betrifft einen medizinischen Saugregulator mit einem Instrumentengehäuse, in dem ein das Instrumentengehäuse in Längsrichtung durchziehender Strömungskanal ausgebildet ist, wobei am distalen und proximalen Ende des Instrumentengehäuses Anschlussstutzen angeordnet sind, über die der Strömungskanal mit einem medizinischen Instrument und einer externen Saug-/Spülvorrichtung koppelbar ist und mit im Instrumentengehäuse ausgebildeten Zuluftöffnung, über die der Strömungskanal quer zur Längsrichtung des Strömungskanals mit der Umgebung strömungstechnisch verbunden ist, wobei die Zufluftöffnungen über ein am Instrumentengehäuse gelagertes Verschlusselement verschließbar sind, wobei im Instrumentengehäuse mehrere in Längsrichtung des Instrumentengehäuses angeordnete Zuluftöffnungen ausgebildet sind und das Verschlusselement zum Verschließen der Zuluftöffnungen als das Instrumentengehäuse koaxial umgebende Hülse ausgebildet ist, die in Längsrichtung des Instrumentengehäuses verlagerbar ist.

Während chirurgischer Operationen ist es häufig erforderlich, Flüssigkeiten, wie beispielsweise Blut oder Spülflüssigkeit, aus dem Operationsgebiet abzusaugen. Hierzu können mit einem Saugkanal ausgestattete chirurgische Instrumente über eine vorzugsweise flexible Saugleitung mit einer externen Saugquelle, beispielsweise einer Vakuumpumpe, verbunden werden.

Da in Operationssälen die Leistung der den Saugstrom erzeugenden Vakuumpumpe in der Regel nicht regulierbar ist, ist es aus der Praxis bekannt, in die Saugleitung, also zwischen das distale chirurgische Instrument und die proximale Saugquelle, einen Saugregulator zu integrieren, über den die Saugleistung regulierbar ist.

Gemäß einer besonders einfachen Ausgestaltung eines derartigen Saugregulators ist es aus der Praxis bekannt, die Regulierung des Saugstroms durch die Saugleitung durch eine Veränderung des Leitungsquerschnitts der Saugleitung zu bewirken. Diese technisch sehr einfach zu bewerkstelligende Art der Saugstromregulierung weist jedoch den Nachteil auf, dass aus dem Operationsgebiet abgesaugte feste Bestandteile, wie beispielsweise Knochenstücke oder Knorpelstücke, sich im reduzierten Querschnitt der Saugleitung verklemmen und die Saugleitung verstopfen können.

Ein gattungsgemäßer Saugregulator ist aus der US 5 084 045 bekannt. Bei diesem bekannten Saugregulator ist die das Verschlusselement bildende Hülse als in Längsrichtung auf das Instrumentengehäuse aufschiebbar ausgebildet. Das reine axiale Aufschieben der Verschlusshülse auf das Instrumentengehäuse ermöglicht es zwar, die Zuluftöffnungen stufenlos zu öffnen und zu schließen, jedoch ist diese Ausgestaltungsform sehr anfällig gegenüber einer versehentlichen Betätigung des Verschlusselements.

Eine weitere Ausgestaltungsform zur Ausbildung eines Saugregulators ist aus der EP 1 553 994 B1 bekannt. Dieser bekannte Saugregulator weist eine im Instrumentengehäuse ausgebildete und quer zur Längsrichtung des Instrumentengehäuses verlaufende längliche Zuluftöffnung auf, über die der Strömungskanal mit der Umgebung strömungstechnisch verbunden ist. Diese Zufluftöffnung ist über ein am Instrumentengehäuse gelagertes Verschlusselement in vorgegebenen Rastschritten verschließbar.

Je weiter die Zuluftöffnung geöffnet ist, desto mehr Nebenluft kann aus der Umgebung angesaugt werden, was zu einer Reduzierung der Saugleistung am distalen Ende der Saugleitung, also am chirurgischen Instrument führt. Umgekehrt erhöht sich die Saugleistung am distalen Ende der Saugleitung je weiter die Zufluftöffnung geschlossen wird und keine Nebenluft aus der Umgebung mehr angesaugt werden kann.

Dieser bekannte Saugregulator ermöglicht zwar eine Regulierung des Saugstroms ohne eine Querschnittsreduzierung der Saugleitung, jedoch ist einerseits die Handhabung des quer zur Instrumentenlängsachse verlagerbaren Verschlusselements unhandlich und andererseits die Öffnung der Zuluftöffnung so groß, dass bei geringer Saugleistung angesaugte Flüssigkeit durch die Zuluftöffnung seitlich aus dem Saugregulator austreten könnte.

Davon ausgehend liegt der Erfindung die **Aufgabe** zugrunde, einen medizinischen Saugregulator zu schaffen, der bei einfachem Aufbau und einfacher Handhabung eine individuelle Regulierung des Saugstroms ermöglicht.

Die **Lösung** dieser Aufgabenstellung ist erfindungsgemäß dadurch gekennzeichnet, dass die das Verschlusselement bildende Hülse in Längsrichtung auf das Instrumentengehäuse aufschraubbar ist.

Bei dieser Ausführungsform bewirkt die Rotationsbewegung der Hülse beim Aufschrauben auf das Instrumentengehäuse gleichzeitig eine Axialverlagerung der Hülse relativ zur Längsrichtung des Instrumentengehäuses und ermöglicht so das Öffnen und Schließen der Zuluftöffnungen. Gegenüber der reinen Axialverschiebbarkeit der Hülse relativ zum Instrumentengehäuse hat das Aufschrauben den Vorteil, dass die Hülse in ihrer jeweiligen Lage relativ zum Instrumentengehäuse gegen ein unbeabsichtigtes Verschieben gesichert ist.

Durch die Anordnung der Zuluftöffnungen in Längsrichtung des Instrumentengehäuses und die entsprechende Ausbildung des Verschlusselements als das Instrumentengeghäuse koaxial umgebende und in Längsrichtung des Instrumentengehäuses verlagerbare Hülse ist es möglich, die Zuluftöffnungen kontinuierlich zu öffnen und zu schließen und so eine individuelle Regulierung der Saugleistung zu gewährleisten.

Gemäß einer praktischen Ausführungsform der Erfindung wird vorgeschlagen, dass die Zuluftöffnungen in mehreren parallel zueinander angeordneten Reihen im Instrumentengehäuse ausgebildet sind. Die Ausbildung mehrerer nebeneinander angeordneter Zuluftöffnungen gewährleistet eine gleichmäßige Luftzufuhr zum Strömungskanal.

Mit einer bevorzugten Ausführungsform der Erfindung wird vorgeschlagen, dass die Zuluftöffnungen in Längsrichtung des Instrumentengehäuses wendelförmig um den Strömungskanal angeordnet ausgebildet sind. Der wendelförmige Versatz der in Längsrichtung des Instrumentengehäuses hintereinander angeordneten Zuluftöffnungen minimiert die Gefahr, dass Flüssigkeit aus dem Strömungskanal nach außen austreten kann, da die gerade Wegstrecke entlang der Innenseite des Strömungskanals bei anliegendem Saugdruck innerhalb des Strömungskanals zu kurz ist, um einen Durchtritt nach außen zu ermöglichen.

Um insbesondere beim Verschließen der Zuluftöffnungen über das Verschlusselement sicherzustellen, dass keine Nebenluft aus der Umgebung mehr über die vom Verschlusselement abgedeckten Zuluftöffnungen in den Strömungskanal angesaugt werden kann, wird mit der Erfindung weiterhin vorgeschlagen, dass die das Verschlusselement bildende Hülse über mindestens ein Dichtungselement gegenüber dem Instrumentengehäuse abgedichtet ist.

Gemäß einer bevorzugten Ausführungsform der Erfindung wird vorgeschlagen, dass die das Verschlusselement bildende Hülse raststufenfrei, also stufenlos kontinuierlich in Längsrichtung des Instrumentengehäuses verlagerbar ist, um so alle möglichen Zwischenstufen des Ganz- oder Fastverschließens bzw. Ganz- oder Fastöffnens der Zuluftöffnungen zu ermöglichen.

Weiterhin wird mit der Erfindung vorgeschlagen, dass die Größe der Zuluftöffnungen so bemessen ist, dass auch bei nur geringen Saugdruck aufgrund der Oberflächenspannung der Flüssigkeit keine Flüssigkeit seitlich aus den Zuluftöffnungen austreten kann, weil das Austreten von Flüssigkeit aus dem Strömungskanal nach außen aus hygienischen Gründen unbedingt verhindert werden sollte. Da in Längsrichtung des Instrumentengehäuses mehrere Zuluftöffnungen angeordnet sind, ist auch ein kleiner Durchmesser der Zuluftöffnungen, wie beispielsweise von kleiner 2mm, ausreichend, um bei geöffnetem Verschlusselement über die Zuluftöffnungen ausreichend Luft ansaugen zu können, um den Saugdruck innerhalb des Strömungskanals zu drosseln.

Schließlich wird mit der Erfindung vorgeschlagen, dass auf der Außenseite der das Verschlusselement bildenden Hülse Griffbereiche, wie beispielsweise in Längsrichtung der Hülse verlaufende Griffmulden, ausgebildet sind, die das Betätigen des Verschlusselements erleichtern.

Weitere Merkmale und Vorteile der Erfindung ergeben sich anhand der zugehörigen Zeichnungen, in denen ein Ausführungsbeispiel eines erfindungsgemäßen medizinischen Saugregulators nur beispielhaft dargestellt ist, ohne die Erfindung auf dieses Ausführungsbeispiel zu beschränken. In den Zeichnungen zeigt:
- Fig. 1: eine perspektivische Seitenansicht eines erfindungsgemäßen medizinischen Saugregulators, den Saugregulator in einer teilweise geöffneten Stellung darstellend;
- Fig. 2: einen Längsschnitt durch den Saugregulator gemäß Fig. 1;
- Fig. 3: eine Schnittdarstellung gemäß Fig. 2, jedoch den Saugregulator der geschlossenen Stellung darstellend und
- Fig. 4: eine Seitenansicht des Instrumentengehäuses ohne Verschlusselement.

Während chirurgischer Operationen ist es häufig erforderlich, Flüssigkeiten, wie beispielsweise Blut oder Spülflüssigkeit, aus dem Operationsgebiet abzusaugen. Hierzu können mit einem Saugkanal ausgestattete chirurgische Instrumente über eine vorzugsweise flexible Saugleitung mit einer externen Saugquelle, beispielsweise einer Vakuumpumpe, verbunden werden.

Da in Operationssälen die Leistung der den Saugstrom erzeugenden Vakuumpumpe in der Regel nicht regulierbar ist, wird in die Saugleitung, also zwischen das distale chirurgische Instrument und die proximale Saugquelle, ein Saugregulator 1 integriert, über den die Saugleistung regulierbar ist.

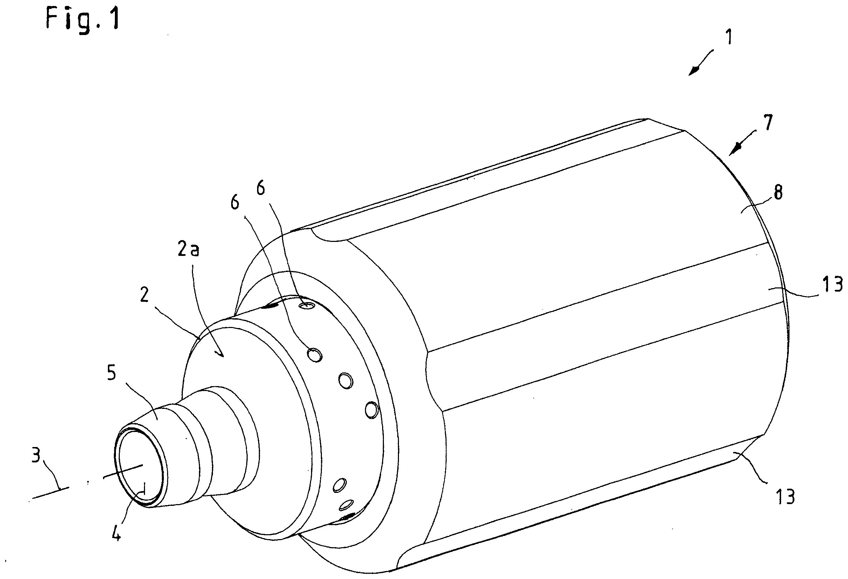

Die Abbildungen Fig. 1 bis Fig. 3 zeigen einen Saugregulator 1 mit einem Instrumentengehäuse 2, in dem ein das Instrumentengehäuse 2 entlang dessen Längsachse 3 durchziehender Strömungskanal 4 ausgebildet ist.

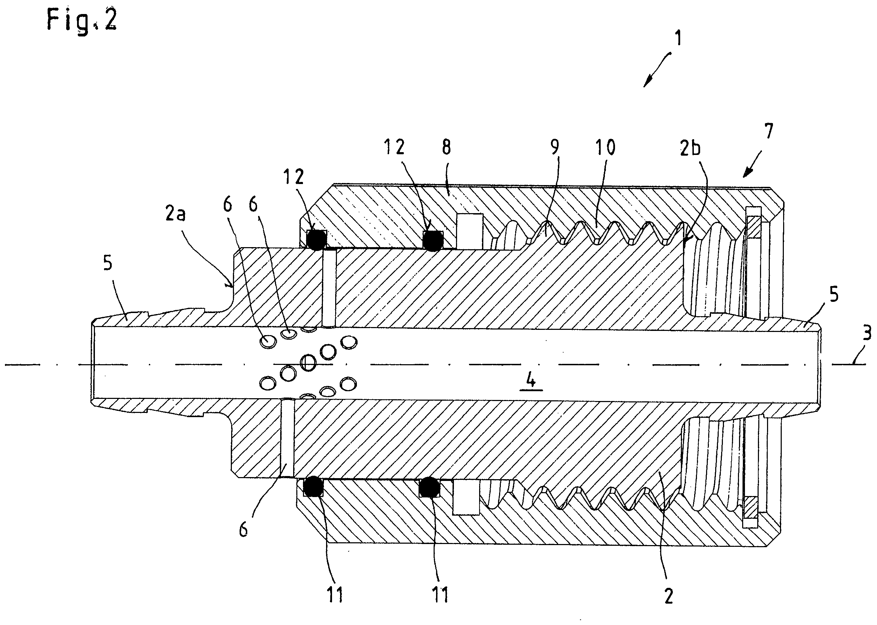

Wie insbesondere aus Fig. 2 bis Fig. 4 ersichtlich, sind am distalen 2a und proximalen 2b Ende des Instrumentengehäuses 2 Anschlussstutzen 5 angeordnet, über die der Strömungskanal 4 mit einem medizinischen Instrument und einer externen Saug-/Spülvorrichtung koppelbar ist.

Um über den Saugregulator 1 die Saugleistung innerhalb des Strömungskanals 4 regulieren zu können, sind im Instrumentengehäuse 2 Zuluftöffnungen 6 ausgebildet, über die der Strömungskanal 4 quer zur Längsrichtung des Strömungskanals 4 mit der Umgebung strömungstechnisch verbunden ist. Die Zuluftöffnungen 6 sind über ein am Instrumentengehäuse 2 gelagertes Verschlusselement 7 verschließbar.

Je weiter die Zuluftöffnungen 6 geöffnet sind oder je mehr Zuluftöffnungen 6 geöffnet sind, desto mehr Nebenluft kann aus der Umgebung angesaugt werden, was zu einer Reduzierung der Saugleistung am distalen Ende des Strömungskanals 4, also am chirurgischen Instrument führt. Umgekehrt erhöht sich die Saugleistung am distalen Ende des Strömungskanals 4, je weiter die Zufluftöffnungen 6 geschlossen werden bzw. je mehr Zuluftöffnungen 6 geschlossen werden und keine Nebenluft aus der Umgebung mehr angesaugt werden kann.

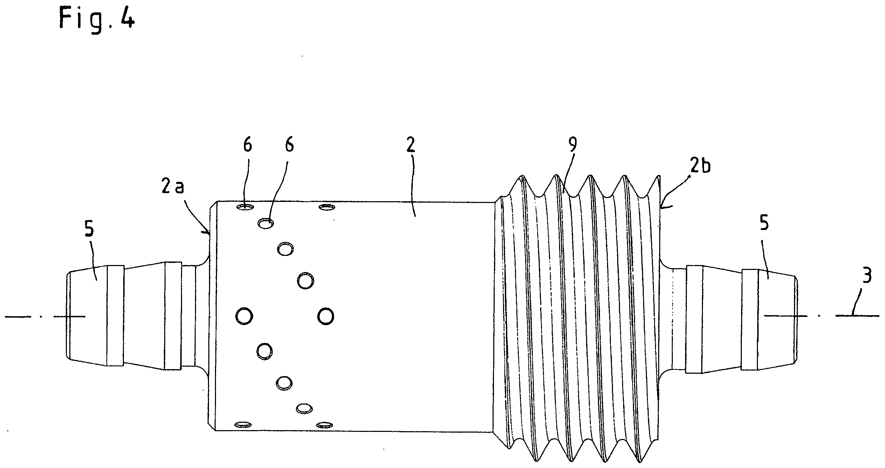

Wie insbesondere aus Fig. 4 ersichtlich, sind im Instrumentengehäuse 2 mehrere in Längsrichtung des Instrumentengehäuses 2 angeordnete Zuluftöffnungen 6 ausgebildet, die in Längsrichtung des Instrumentengehäuses 2 wendelförmig um den Strömungskanal 4 angeordnet sind.

Das Verschlusselement 7 ist bei der dargestellten Ausführungsform als das Instrumentengehäuse 2 koaxial umgebende Hülse 8 ausgebildet, die in Längsrichtung auf das Instrumentengehäuse 2 aufschraubbar ist. Zum Aufschrauben der Hülse 8 auf das Instrumentengehäuse 2 weist das proximale Ende des Instrumentengehäuses 2 ein Außengewinde 9 auf, das mit einem Innengewinde 10 auf der Innenseite der Hülse 8 korrespondiert.

Die Zuluftöffnungen 6 sind im gewindefreien distalen Teil des Instrumentengehäuses 2 ausgebildet, wie dies den Abbildungen Fig. 2 bis Fig. 4 zu entnehmen ist.

Um insbesondere beim Verschließen der Zuluftöffnungen 6 über das als Hülse 8 ausgebildete Verschlusselement 7 sicherzustellen, dass keine Nebenluft aus der Umgebung mehr über die bereits von der Hülse 8 abgedeckten Zuluftöffnungen 6 in den Strömungskanal 4 angesaugt werden kann, ist die Hülse 8 über Dichtungselemente 11 gegenüber dem Instrumentengehäuse 2 abgedichtet.

Bei der dargestellten Ausführungsform sind zwei als O-Ringe ausgebildete Dichtungselemente 11 vorgesehen, die in umlaufenden Nuten 12 angeordnet sind, die in Axialrichtung der Hülse 8 voneinander beabstandet, in der dem Instrumentengehäuse 2 zugewandten Innenseite der Hülse 8 ausgebildet sind.

Auf der Außenseite der das Verschlusselement 7 bildenden Hülse 8 sind Griffbereiche in der Form in Längsrichtung der Hülse 8 verlaufender Griffmulden 13 ausgebildet, die das Betätigen des Verschlusselements 7 erleichtern. Gemäß alternativer Ausführungsformen können die Griffbereiche beispielsweise auch als Aufrauhungen der Oberfläche oder punktförmige Erhebungen oder Vertiefungen ausgebildet sein.

Ein wie zuvor beschrieben ausgebildeter Saugregulator 1 ist wie folgt zu bedienen:
Über die Anschlussstutzen 5 wird der Saugregulator distalseitig 2a mittels eines Schlauchs mit der Saugleitung eines chirurgischen Instruments und proximalseitig 2b mittels eines Schlauchs mit einer externen Saugvorrichtung, wie beispielsweise einer Vakuumpumpe, verbunden.

Je nachdem, wie stark die Saugleistung am distalen Ende des chirurgischen Instruments sein soll, lässt sich über den Saugregulator 1 durch Öffnen oder Verschließen der Zuluftöffnungen 6 die Saugleistung regulieren.

Die Abbildungen Fig. 1 und 2 zeigen den Saugregulator 1 in einer Stellung, in der drei von fünf der in Axialrichtung wendelförmig hintereinander angeordneten Zuluftöffnungen 6 geöffnet sind. In dieser Stellung wird über die offenen, also nicht durch das Verschlusselement 7 abgedeckten Zuluftöffnungen 6 Nebenluft aus der Umgebung angesaugt, was bei konstanter Leistung der externen proximalseitigen Saugvorrichtung zu einer Reduzierung der Saugleistung am distalen Ende des Strömungskanals 4, also am chirurgischen Instrument führt.

Wenn jetzt die Saugleistung innerhalb des Strömungskanals 4 und somit am chirurgischen Instrument erhöht werden soll, schraubt der Operateur die das Verschlusselement 7 bildende Hülse 8 weiter in die distale Richtung auf das Instrumentengehäuse 2 auf, wodurch mehr und mehr Zuluftöffungnen 6 verschlossen werden.

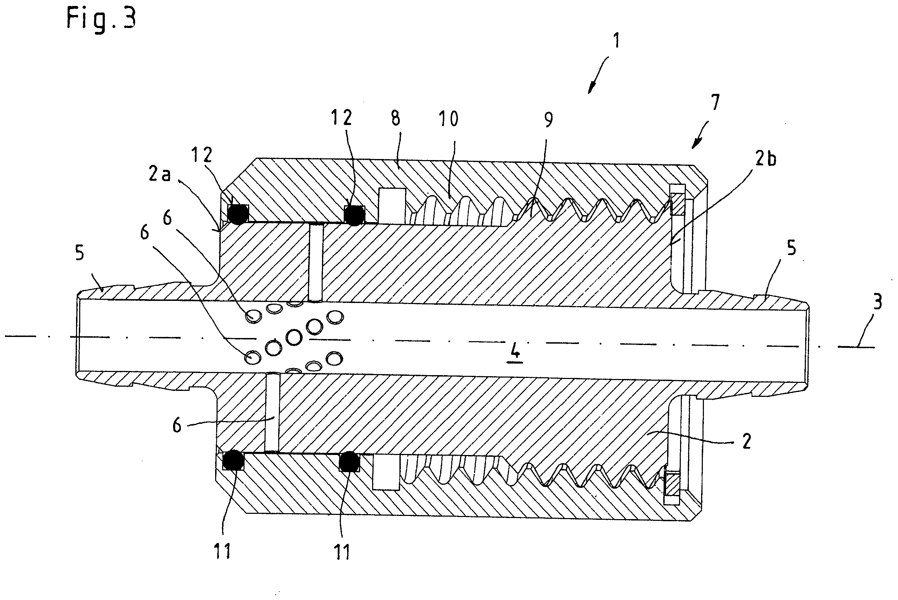

Die Abbildung Fig. 3 zeigt den Saugregulator 1 in der vollständig geschlossenen Position, in der alle Zuluftöffnungen 6 über das Verschlusselement 7 verschlossen sind. In dieser Stellung kann keine Nebenluft über die Zuluftöffnungen 6 angesaugt werden, so dass die volle Saugleistung der externen Saugvorrichtung im Strömungskanal 4 und somit am chirurgischen Instrument anliegt.

Die Ausbildung des Verschlusselements 7 als auf das Instrumentengehäuse 2 aufschraubbare Hülse 8 hat den Vorteil, dass die Saugleistung stufenlos kontinuierlich regulierbar ist. Die Ausbildung der Griffmulden 13 auf der Außenseite der Hülse 8 vereinfacht die Bedienung insbesondere beim Einhandbetrieb des Saugregulators 1.

Gegenüber der reinen Axialverschiebbarkeit der Hülse 8 relativ zum Instrumentengehäuse 2 hat das Aufschrauben aber den Vorteil, dass die Hülse 8 beim Aufschrauben in ihrer jeweiligen Lage relativ zum Instrumentengehäuse 2 gegen ein unbeabsichtigtes Verschieben gesichert ist.

Die Größe der Zuluftöffnungen 6 ist so bemessen, dass auch bei nur geringen Saugdruck aufgrund der Oberflächenspannung der Flüssigkeit keine Flüssigkeit seitlich aus den Zuluftöffnungen 6 austreten kann, weil das Austreten von Flüssigkeit aus dem Strömungskanal 4 nach außen aus hygienischen Gründen unbedingt verhindert werden sollte. Da in Längsrichtung des Instrumentengehäuses 2 mehrere Zuluftöffnungen 6 angeordnet sind, ist auch ein kleiner Durchmesser der Zuluftöffnungen 6 von beispielsweise kleiner 2mm ausreichend, um bei geöffnetem Verschlusselement 7 über die Zuluftöffnungen 6 ausreichend Luft ansaugen zu können, um den Saugdruck innerhalb des Strömungskanals 4 zu drosseln.

Ein wie zuvor beschrieben ausgebildeter Saugregulator 1 hat den Vorteil, dass bei einfachem Aufbau und einfacher Handhabung eine individuelle und kontinuierliche Regulierung des Saugstroms möglich ist.

### Bezugszeichenliste

- 1: Saugregulator
- 2: Instrumentengehäuse
- 2a: distales Ende
- 2b: proximales Ende
- 3: Längsachse
- 4: Strömungskanal
- 5: Anschlussstutzen
- 6: Zuluftöffnung
- 7: Verschlusselement
- 8: Hülse
- 9: Außengewinde
- 10: Innengewinde
- 11: Dichtungselement
- 12: Nut
- 13: Griffmulde

## Patentansprüche

1. Medizinischer Saugregulator mit einem Instrumentengehäuse (2), in dem ein das Instrumentengehäuse (2) in Längsrichtung durchziehender Strömungskanal (4) ausgebildet ist, wobei am distalen (2a) und proximalen (2b) Ende des Instrumentengehäuses (2) Anschlussstutzen (5) angeordnet sind, über die der Strömungskanal (4) mit einem medizinischen Instrument und einer externen Saug-/Spülvorrichtung koppelbar ist und mit im Instrumentengehäuse (2) ausgebildeten Zuluftöffnung (6), über die der Strömungskanal (4) quer zur Längsrichtung des Strömungskanals (4) mit der Umgebung strömungstechnisch verbunden ist, wobei die Zufluftöffnungen (6) über ein am Instrumentengehäuse (2) gelagertes Verschlusselement (7) verschließbar sind, wobei im Instrumentengehäuse (2) mehrere in Längsrichtung des Instrumentengehäuses (2) angeordnete Zuluftöffnungen (6) ausgebildet sind und das Verschlusselement (7) zum Verschließen der Zuluftöffnungen (6) als das Instrumentengehäuse (2) koaxial umgebende Hülse (8) ausgebildet ist, die in Längsrichtung des Instrumentengehäuses (2) verlagerbar ist,
**dadurch gekennzeichnet,**
**dass** die das Verschlusselement (7) bildende Hülse (8) in Längsrichtung auf das Instrumentengehäuse (2) aufschraubbar ist.

2. Medizinischer Saugregulator nach Anspruch 1, **dadurch gekennzeichnet, dass** die Zuluftöffnungen (6) in mehreren parallel zueinander angeordneten Reihen im Instrumentengehäuse (2) ausgebildet sind.

3. Medizinischer Saugregulator nach Anspruch 1 oder 2, **dadurch gekennzeichnet, dass** die Zuluftöffnungen (6) in Längsrichtung des Instrumentengehäuses (2) wendelförmig um den Strömungskanal (4) angeordnet ausgebildet sind.

4. Medizinischer Saugregulator nach einem der Ansprüche 1 bis 3, **dadurch gekennzeichnet, dass** die das Verschlusselement (7) bildende Hülse (8) über mindestens ein Dichtungselement (11) gegenüber dem Instrumentengehäuse (2) abgedichtet ist.

5. Medizinischer Saugregulator nach einem der Ansprüche 1 bis 4, **dadurch gekennzeichnet, dass** die das Verschlusselement (7) bildende Hülse (8) stufenlos in Längsrichtung des Instrumentengehäuses (2) verlagerbar ist.

6. Medizinischer Saugregulator nach einem der Ansprüche 1 bis 5, **dadurch gekennzeichnet, dass** die Größe der Zuluftöffnungen (6) so bemessen ist, dass auch bei nur geringen Saugdruck aufgrund der Oberflächenspannung der Flüssigkeit keine Flüssigkeit seitlich aus den Zuluftöffnungen (6) austreten kann.

7. Medizinischer Saugregulator nach einem der Ansprüche 1 bis 6, **dadurch gekennzeichnet, dass** auf der Außenseite der das Verschlusselement (7) bildenden Hülse (8) Griffbereiche ausgebildet sind.

8. Medizinischer Saugregulator nach Anspruch 7, **dadurch gekennzeichnet, dass** die Griffbereiche als in Längsrichtung der Hülse (8) verlaufende Griffmulden (13) ausgebildet sind.

## Claims

1. A medical suction regulator having an instrument housing (2), in which a flow channel (4) passing into the instrument housing (2) in the longitudinal direction is formed, wherein, at the distal (2a) and proximal (2b) ends of the instrument housing (2), connecting pieces (5) are arranged, by means of which the flow channel (4) can be connected to a medical instrument and an external suction/flushing device, and having a supply air opening (6) formed in the instrument housing (2), by means of which the flow channel (4) is fluidically connected to the environment transverse to the longitudinal direction of the flow channel (4), wherein the supply air openings (6) can be closed off by means of a closure element (7) mounted on the instrument housing (2), wherein multiple supply air openings (6) arranged in the longitudinal direction of the instrument housing (2) are formed in the instrument housing (2), and the closure element (7) for closing off the supply air openings (6) is formed as the sleeve (8) coaxially surrounding the instrument housing (2), with the sleeve being moveable in the longitudinal direction of the instrument housing (2),
**characterized in that**
the sleeve (8) forming the closure element (7) can be screwed onto the instrument housing (2) in the longitudinal direction.

2. The medical suction regulator according to claim 1, **characterized in that** the supply air openings (6) are formed in multiple rows arranged parallel to one another in the instrument housing (2).

3. The medical suction regulator according to either claim 1 or 2, **characterized in that** the supply air openings (6) are formed in the shape of a helix arranged around the flow channel (4) in the longitudinal direction of the instrument housing (2).

4. The medical suction regulator according to any one of claims 1 to 3, **characterized in that** the sleeve (8) forming the closure element (7) is sealed off against the instrument housing (2) by means of at least one sealing element (11).

5. The medical suction regulator according to any one of claims 1 to 4, **characterized in that** the sleeve (8) forming the closure element (7) can be moved steplessly in the longitudinal direction of the instrument housing (2).

6. The medical suction regulator according to any one of claims 1 to 5, **characterized in that** the size of the supply air openings (6) is dimensioned such that no fluid can escape laterally from the supply air openings (6), even under low suction pressure, due to the surface tension of the fluid.

7. The medical suction regulator according to any one of claims 1 to 6, **characterized in that** gripping areas are formed on the outer side of the sleeve (8) forming the closure element (7).

8. The medical suction regulator according to claim 7, **characterized in that** the gripping areas are formed as gripping recesses (13) extending in the longitudinal direction of the sleeve (8).

## Revendications

1. Régulateur d'aspiration médical comprenant un boîtier d'instrument (2), dans lequel est formé un canal d'écoulement (4) traversant le boîtier d'instrument (2) dans la direction longitudinale, dans lequel des tubulures de raccordement (5) sont agencées à l'extrémité distale (2a) et proximale (2b) du boîtier d'instrument (2), tubulures par l'intermédiaire desquelles le canal d'écoulement (4) peut être accouplé à un instrument médical et à un dispositif d'aspiration/de rinçage externe, le régulateur comprenant également une ouverture d'arrivée d'air (6), formée dans le boîtier d'instrument (2), par l'intermédiaire de laquelle le canal d'écoulement (4) est en liaison fluidique avec l'environnement transversalement à la direction longitudinale du canal d'écoulement (4), dans lequel les ouvertures d'arrivée d'air (6) peuvent être fermées par un élément de fermeture (7) monté sur le boîtier d'instrument (2), dans lequel plusieurs ouvertures d'arrivée d'air (6) situées dans la direction longitudinale du boîtier d'instrument (2) sont formées dans le boîtier d'instrument (2) et l'élément de fermeture (7) destiné à fermer les ouvertures d'arrivée d'air (6) est réalisé sous la forme d'un manchon (8) qui entoure le boîtier d'instrument (2) de manière coaxiale et qui peut être déplacé dans la direction longitudinale du boîtier d'instrument (2),
**caractérisé**
**en ce que** le manchon (8) formant l'élément de fermeture (7) peut être vissé sur le boîtier d'instrument (2) dans la direction longitudinale.

2. Régulateur d'aspiration médical selon la revendication 1, **caractérisé en ce que** les ouvertures d'arrivée d'air (6) sont formées dans le boîtier d'instrument (2) en plusieurs rangées parallèles les unes aux autres.

3. Régulateur d'aspiration médical selon la revendication 1 ou 2, **caractérisé en ce que** les ouvertures d'arrivée d'air (6) sont formées de manière à être situées de façon hélicoïdale autour du canal d'écoulement (4) dans la direction longitudinale du boîtier d'instrument (2).

4. Régulateur d'aspiration médical selon l'une quelconque des revendications 1 à 3, **caractérisé en ce que** le manchon (8) formant l'élément de fermeture (7) est étanchéifié par rapport au boîtier d'instrument (2) par l'intermédiaire d'au moins un élément d'étanchéité (11).

5. Régulateur d'aspiration médical selon l'une quelconque des revendications 1 à 4, **caractérisé en ce que** le manchon (8) formant l'élément de fermeture (7) peut être déplacé en continu dans la direction longitudinale du boîtier d'instrument (2).

6. Régulateur d'aspiration médical selon l'une quelconque des revendications 1 à 5, **caractérisé en ce que** la taille des ouvertures d'arrivée d'air (6) est dimensionnée de sorte qu'aucun liquide ne peut sortir latéralement des ouvertures d'arrivée d'air (6), y compris lorsque la pression d'aspiration est minime du fait de la tension superficielle du liquide.

7. Régulateur d'aspiration médical selon l'une quelconque des revendications 1 à 6, **caractérisé en ce que** des zones de préhension sont formées sur la face extérieure du manchon (8) formant l'élément de fermeture (7).

8. Régulateur d'aspiration médical selon la revendication 7, **caractérisé en ce que** les zones de préhension sont réalisées sous la forme de poignées encastrées (13) s'étendant dans la direction longitudinale du manchon (8).
